# EUROPEAN PATENT SPECIFICATION

(11) **EP 1 283 048 B1**
(45) Date of publication and mention of the grant of the patent: **02.08.2006**
(21) Application number: 01127056.8
(22) Date of filing: 14.11.2001
(51) Int. Cl.: A61K 36/52, A61K 31/35, A61K 31/70, A61P 13/00

(54) **Therapeutic agent for improving bladder function or treating urinary disturbance and food and drink containing it**
Therapeutischer Wirkstoff zur Verbesserung der Blasenfunktion oder Behandlung von Harnwegstörung und diesen enthaltendes Nahrungsmittel oder Getränk
Agent thérapeutique pour l'amélioration de la fonction vésicale ou le traitement de perturbations urinaires et produit alimentaire ou boisson le contenant

(30) Priority: 09.08.2001 JP 2001242761
(43) Date of publication of application: 12.02.2003
(73) Proprietor: Maruzen Pharmaceuticals Co., Ltd., Onomichi-shi, Hiroshima (JP)
(72) Inventor: Kawashima, Yoshihito, c/o Maruzen Pharma. Co., Ltd, Onomichi-shi, Hiroshima (JP); Mizutani, Kenji, c/o Maruzen Pharma. Co., Ltd., Onomichi-shi, Hiroshima (JP); Levin, Robert M., Albany, NY (US)
(74) Representative: Winter, Brandl, Fürniss, Hübner Röss, Kaiser, Polte Partnerschaft Patent- und Rechtsanwaltskanzlei

(56) References cited:
- DATABASE WPI Section Ch, Week 199551 Derwent Publications Ltd., London, GB; Class D13, AN 1995-399227 XP002219323 & JP 07 274832 A (MARUZEN SEIYAKU KK), 24 October 1995 (1995-10-24)
- PATENT ABSTRACTS OF JAPAN vol. 1997, no. 06, 30 June 1997 (1997-06-30) & JP 09 030984 A (MARUZEN PHARMACEUT CO LTD), 4 February 1997 (1997-02-04)
- PATENT ABSTRACTS OF JAPAN vol. 018, no. 307 (C-1211), 13 June 1994 (1994-06-13) & JP 06 065074 A (MARUZEN PHARMACEUT CO LTD), 8 March 1994 (1994-03-08)
- HARAGUCHI H ET AL: "INHIBITION OF ALDOSE REDUCTASE BY DIHYDROFLAVONOLS IN ENGELHARDTIA CHRYSOLEPIS AND EFFECTS ON OTHER ENZYMES" EXPERIENTIA, BIRKHAUSER VERLAG. BASEL, CH, vol. 52, no. 6, 15 June 1996 (1996-06-15), pages 564-567, XP000983720 ISSN: 0014-4754
- MIZUTANI K ET AL: "ANTITUMOR-PROMOTING ACTIVITIES OF DIHYDROFLAVONOLS FROM KOHKI TEA, THE LEAVES OF ENGELHARDTIA CHRYSOLEPIS" FOOD FACTORS FOR CANCER PREVENTION, XX, XX, 1997, pages 607-612, XP000983752
- MOTOYASHIKI T ET AL: "ENHANCEMENT OF THE VANADATE-STIMULATED RELEASE OF LIPOPROTEIN LIPASE ACTIVITY BY STILBIN FROM THE LEAVES OF ENGELHARDTIA CHRYSOLEPIS" BIOLOGICAL & PHARMACEUTICAL BULLETIN (OF JAPAN), PHARMACEUTICAL SOCIETY OF JAPAN, JP, vol. 21, no. 5, 1998, pages 517-519, XP000942125 ISSN: 0918-6158

## Description

### Technical Field

The present invention relates to a safe and effective therapeutic agent for improving bladder function or treating urinary disturbance which is useful for improving bladder function as well as preventing/treating urinary disturbance, and to food and drink for improving bladder function or treating urinary disturbance.

The term "improving bladder function" herein means treating bladder dysfunction associated with urinary disturbance caused by, for example, prostatomegaly. The term "treating urinary disturbance" herein includes: reducing frequency of micturition in pollakiuria; ameliorating nycturia; increasing urinary pressure and quantity per one miction; eliminating dribbling after urination; eliminating difficulty in initiating miction; reducing residual or retained urine; or treating urinal incontinence in which micturition desire may be lost. The term "food and drink" herein include a wide range of general foods, health foods, supplement foods, and quasi and pharmaceutical drugs which can be orally administered.

### Background of the Invention

Diseases which may lead to urinary disturbance may include those caused by disorders of prostate, bladder or urinary tract as well as neurogenic disorders. Among all, prostatomegaly or prostatic hypertrophy is now increasing with progressive increase in elderly population and, at the present stage, some treatments (particularly radical cures such as surgical treatment and drug therapy) have certain effects on it.

However, prostatomegaly at early stage (i.e., slight prostatomegaly prior to dysfunction) is likely to be overlooked since it may not exhibit any distinct symptom. Thus, reduced bladder dysfunction caused by early stage prostatomegaly may result in various urinary disturbances such as reduced urinary pressure, dribbling after urination, or difficulty in initiating miction.

Further, since any fundamental therapy for bladder dysfunction has not been established yet, once bladder dysfunction is caused by prostatomegaly, it may be difficult, in many cases, to ameliorate the reduced bladder function even when prostatomegaly is treated. Therefore, many cases have been reported that urinary disturbances could not be ameliorated due to the reduced bladder function. Moreover, by facilitating regulation of bladder function may allow for conservative treatment, needs of means for improving bladder function is widely recognized.

However, conventional treatments of bladder dysfunction have been addressed only to prostate by administration of drug which may decrease the size of prostate such as 5α-reductase inhibitor such as finasteride or postatic smooth muscle α-adrenergic receptor inhibitor. Therefore, these treatments have been insufficient to prevent and treat bladder dysfunction and/or urinary disturbances.

### Disclosure of the Claimed Invention

Purpose of the present invention is to overcome the disadvantages in the prior art and to achieve the following objects.

In summary, one object of the present invention is to provide a use of an extract of Engelhardtia chrysolepis for the production of a safe and effective therapeutic agent for improving bladder function or treating urinary disturbance. Another object of the present invention is to provide the use of an extract of Engelhardtia chrysolepis for the production of a food and drink for improving bladder function or treating urinary disturbance which contains such therapeutic agent.

### Technical Problems, Means for Solving the Problems and Effect of the Invention

The present inventors found, after intense studies, that an extract of *Engelhardtia chrysolepis* is very effective in improving bladder function and treating urinary disturbance.

The present inventors also found that food and drink which may be orally administered daily (including general foods, health foods, supplement foods, quasi and pharmaceutical drugs) that contain the therapeutic agent for improving bladder function or treating urinary disturbance according to the present invention may be effective in treating bladder dysfunction associated with urinary disturbances caused by prostatomegaly as well as that in prevention and treatment of urinary disturbances by, for example: reducing frequency of micturition in pollakiuria; ameliorating nycturia; increasing urinary pressure and quantity per single miction; eliminating dribbling after urination; eliminating difficulty in initiating miction; reducing residual or retained urine; or treating urinal incontinence in which micturition desire may be lost. The present invention is based on these findings.

In summary, the present invention provides the use of an extract of Engelhardtia chrysolepis for the production of a therapeutic agent for improving bladder function or treating urinary disturbance, and the use of an extract of Engelhardtia chrysolepis for the production of food and drink which are effective in improving bladder function or treating urinary disturbance, which will be described below.

The first aspect of the invention provides the use of an extract of Engelhardtia chrysolepis for the production of therapeutic agent for improving bladder function or treating urinary disturbance.

The second aspect of the invention provides the use according to claim 1, wherein the extract is obtained by extracting the leaves and/or stalk of *Engelhardtia chrysolepis* or the whole *Engelhardtia chrysolepis* with water, hydrophilic organic solvent or mixture thereof.

The invention according to claim 3 provides the use according to the first or the second aspect, wherein the extract is obtained by extracting leaves of *Engelhardtia chrysolepis* with water, ethyl alcohol or mixture thereof.

A fourth aspect (not comprised by the present invention) provides a therapeutic agent for improving bladder function or treating urinary disturbance which contains, as active ingredient, one or more selected from the group consisting of dihydroflavonols of the following formula (1): 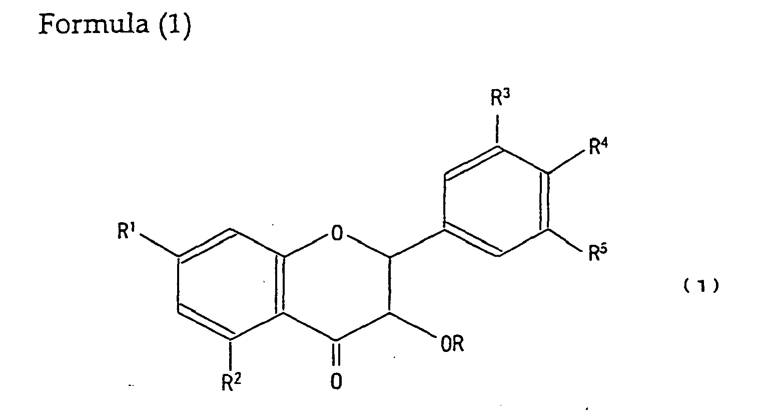 [wherein R represents hydrogen, monosaccharide or disaccharide group, and R¹-R⁵ independently represent hydrogen or hydroxyl group]
and glycosides thereof.

A fifth aspect (not comprised by the present invention) provides a therapeutic agent according to the fourth aspect characterized in that the active ingredient of formula (1) above is selected from the group consisting of astilbin, neoisoastilbin, isoastilbin, neoastilbin, taxifolin, or any combination thereof.

A sixth aspect (not comprised by the present invention) provides a therapeutic agent according to the fourth aspect or the fifth aspect characterized in that the active ingredient of formula (1) above is obtained by purifying an extract of one or more selected from the group consisting of *Engelhardtia chrysolepis, Lyonia ovalifolia, Chloranthus glaber, Smilax glabra, Astilbe microphylla* and *Astilbe odontophylla.*

A seventh aspect (not comprised by the present invention) provides a therapeutic agent according to the sixth aspect characterized in that the active ingredient of formula (1) above is obtained by purifying an extract of leaves of *Engelhardtia chrysolepis* obtained by extraction with water, ethyl alcohol or mixture thereof.

The eighth aspect of the invention provides the use of an extract of Engelhardtia chrysolepis for the production of a food and drink effective in improving bladder function or treating urinary disturbance, wherein the active ingredient is obtained in accordance with any of the first aspect through the third aspect.

The ninth aspect of the invention provides the use of food and drink-according to the eighth aspect characterized in that the active ingredient recited in any of the first aspect through the seventh aspect is present in an amount of 0.01-50% by weight of the total food or drink.

The tenth aspect of the invention provides the use according to the eighth aspect characterized in that the active compounds recited in any one of the first aspect through the third aspect is present in an amount of 0.1-100% by weight of the total food or drink and wherein the food or drink being provided in a form of granule, tablet or capsule.

*Engelhardtia chrysolepis* has been used for more than one thousand years as very common and safe folk medicine, and particularly taken as tea. The present applicant previously suggested, after intense study, that an extract of *Engelhardtia chrysolepis* can preferably be used as inhibitor of cancer promotion (Japanese Patent Application Laid-Open No. 6-247851), inhibitor of aldose reductase (a rate-limiting enzyme occurring in sorbitol pathway)(Japanese Patent Application Laid-Open No. 9-30984), active oxygen eliminator (Japanese Patent Application Laid-Open No. 6-65074), anti-inflammatory agent (Japanese Patent Application Laid-Open No. 6-256194), skin-whitening agent and external application for skin (Japanese Patent Application Laid-Open No. 7-223933), bath salts or other applications (Japanese Patent Application Laid-Open No. 8-277214) or the like.

However, the present inventors were the first to find that if an extract of *Engelhardtia chrysolepis,* and also certain dihydroflavonols and/or glycosides thereof, although not comprised by the present invention, are used as active ingredient for the production of a therapeutic agent, tian trés/_agent have certain ability to treat bladder dysfunction associated with urinary disturbances caused by prostatomegaly, and may be very effective in prevention and treatment of urinary disturbances by, for example: reducing frequency of micturition in pollakiuria; ameliorating nycturia; increasing urinary pressure and quantity per single miction; eliminating dribbling after urination; eliminating difficulty in initiating miction; reducing residual or retained urine; or treating urinal incontinence in which micturition desire may be lost.

The present invention can provide a use of an extract of Engelhardtia chrysolepis for the production of a safe therapeutic agent for improving bladder function or treating urinary disturbance which contains, as active ingredient the extract of *Engelhardtia chrysolepis.*

The present invention may also provide a use of an extract of Engelhardtia chrysolepis for the productive of food and drink for improving bladder function or treating urinary disturbance which can easily be taken everyday. Such food and drink are very effective in treating bladder dysfunction associated with urinary disturbance caused by prostatomegaly, as well as in prevention and/or treatment of urinary disturbance by, for example; reducing frequency of micturition in pollakiuria; ameliorating nycturia; increasing urinary pressure and quantity per single miction; eliminating dribbling after urination; eliminating difficulty in initiating miction; reducing residual or retained urine; or treating urinal incontinence in which micturition desire may be lost.

### Brief Description of Drawings

Figure 1 is a graph showing the intravesical pressure at 20% vesical capacity in Example 1.
Figure 2 is a graph showing the intravesical pressure at 100% vesical capacity in Example 1.
Figure 3 is a graph showing the maximum contractive force in response to electric or carbachol stimulation in Example 2.
Figure 4 is a graph showing Ca²⁺-dependent ATPase activity in ladder muscle in Example 3.
Figure 5 is a graph showing Ca²⁺-dependent ATPase activity in ladder mucosa in Example 3.
Figure 6 is a graph showing Citrate Synthase activity in Example 3.

### Detailed Description of the Embodiments

Detailed description of the present invention will be provided below.

Therapeutic agents for improving bladder function or treating urinary disturbance be obtained by using an extract of *Engelhardtia chrysolepis* as the active ingredient according to one aspect of the present invention.

*Engelhardtia chrysolepis* is an evergreen tree which can be found wild in southern part of China. *Engelhardtia chrysolepis* extraction is also called "sweet taste tea" which has been taken for long time for the purpose of antipyresis, detoxication, slimming or the like.

Particularly, extract that is used according to the present invention may preferably be obtained from the parts above the ground (e.g., leaves and stalks) of *Engelhardtia chrysolepis*, and more preferably from the leaves of *Engelhardtia chrysolepis* in terms of its physiological activity though the whole *Engelhardtia chrysolepis* tree can be used. The term "an extract of *Engelhardtia chrysolepis"* herein includes solution of an extract of *Engelhardtia chrysolepis*, diluted or concentrated solution of such extract, dry product obtained by drying such solution of extract, and crude or purified product thereof.

Therapeutic agents for improving bladder function or treating urinary disturbance prepared by the use according to the present invention can be obtained by immersing the leaves and/or stalks of *Engelhardtia chrysolepis* or the whole *Engelhardtia chrysolepis* in water, hydrophilic organic solvent or mixture thereof, and extracting components therefrom at a temperature of a range from room temperature to the boiling point of the solvent used in any suitable device.

Water which can be used as extraction solution includes pure water, tap water, well water, mineral spring water, mineral water, hot-spring water, spring water, freshwater and processed waters. Processed waters may include those processed by, for example, purification, heating, disinfection, sterilization, filtration, ion-exchange method, osmotic pressure adjustment, treatment with buffer or the like. Therefore, water which can be used as extraction solvent in the present invention also includes purified water, hot water, ion-exchanged water, physiological saline, phosphate buffer and phosphate buffered saline (PBS).

Examples of said hydrophilic organic solvent include, for example: C1-C5 lower alcohol such as methanol, ethanol, propyl alcohol or isopropyl alcohol; lower aliphatic ketone such as acetone or methyl ethyl ketone; and C2-C5 polyalcohol such as 1,3-butylene glycol, propylene glycol, isoprene glycol or glycerin. Mixture of water and such hydrophilic organic solvent(s) may be used. Such mixture of water and hydrophilic organic solvent(s) may be preferably contain, for example: for lower alcohol, 1-90 parts against 10 parts of water; for lower aliphatic ketone, 1-40 parts against 10 parts of water; or for polyalcohol, 10-90 parts against 10 parts of water.

Particularly, crushed dry leaves of *Engelhardtia chrysolepis* may be loaded in a tank filled with extraction solvent and then left to stand for a period of time of from 30 minutes to 2 hours while occasionally stirring when required, thereby allowing soluble components to be eluted into the solvent. Next, the solution may be filtered to remove solid materials and then distilled to remove solvent. The resultant wet product may be then dried to obtain solid extract. Typically, solvent: *Engelhardtia chrysolepis* = 5-15:1 (wt%) may be preferable. Extraction may typically be performed at 50-95°C for about 1-4 hours when water is used as solvent, or at 40-80°C for about 30 minutes to 4 hours when mixture of water and ethanol (e.g., ethanol containing 30-80wt% water) is used.

Solvent may be removed by distillation from the filtered or centrifuged solution of extract to give paste of enriched extract. The paste may be dried to obtain solid extract. Extract of *Engelhardtia chrysolepis* according to the present invention may be provided in a solid form or as a solution or enriched solution of the extract. Extract may be used after purification by treatment with activated carbon, adsorptive resin or ion-exchange resin, or by liquid-liquid countercurrent distribution method provided that the object of the present invention may not be prevented.

Next, although not comprised by the present invention, therapeutic agents for improving bladder function or treating urinary disturbance contain, as active ingredient, one or more selected from the group consisting of dihydroflavonols of the following formula (1): 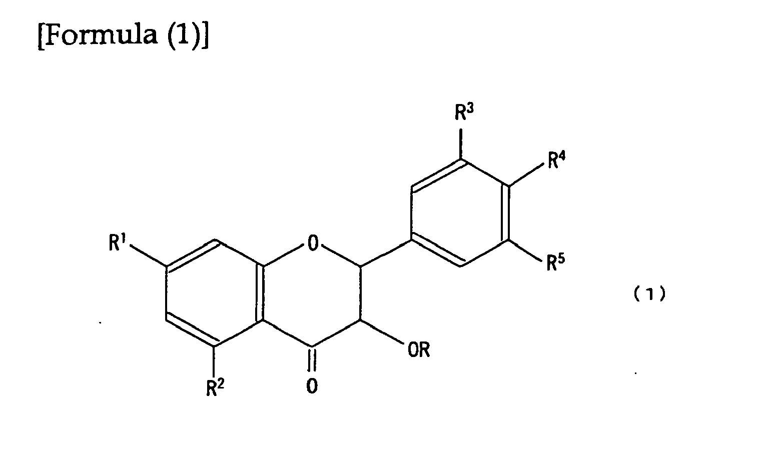 [wherein R represents hydrogen, monosaccharide or disaccharide group such as a -L-rhamnose or 3-O-β-D-glucosyl- α-L-rhamnose group, and R¹-R⁵ independently represent hydrogen or hydroxyl group]
and glycosides thereof.

Preferable dihydroflavonols of formula (1) above and glycosides thereof include astilbin, neoisoastilbin, isoastilbin, neoastilbin (those are hereinafter referred to as "astilbins"), taxifolin, represented by formulae shown below, or any combination thereof.

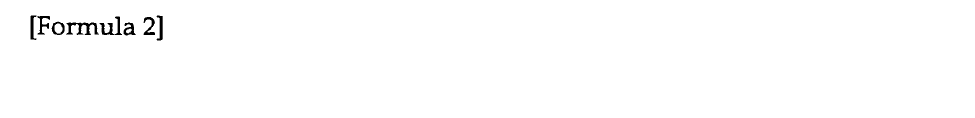

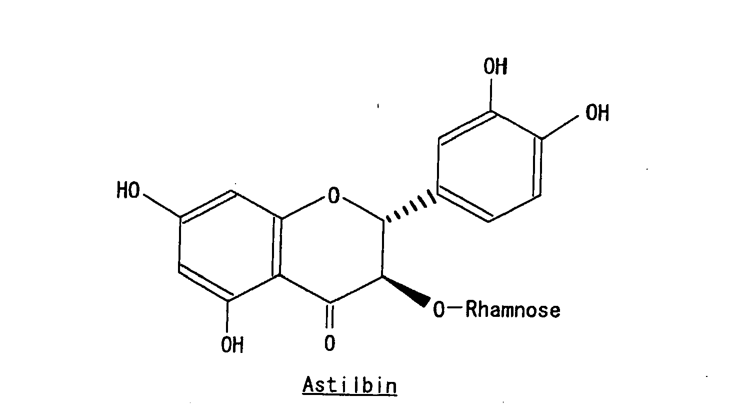

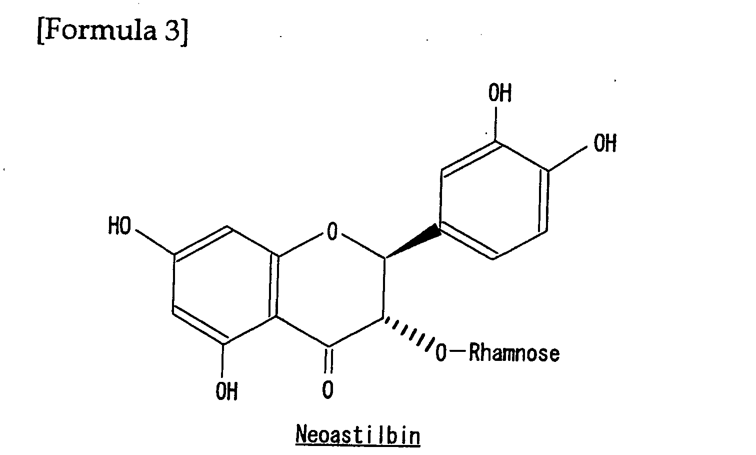

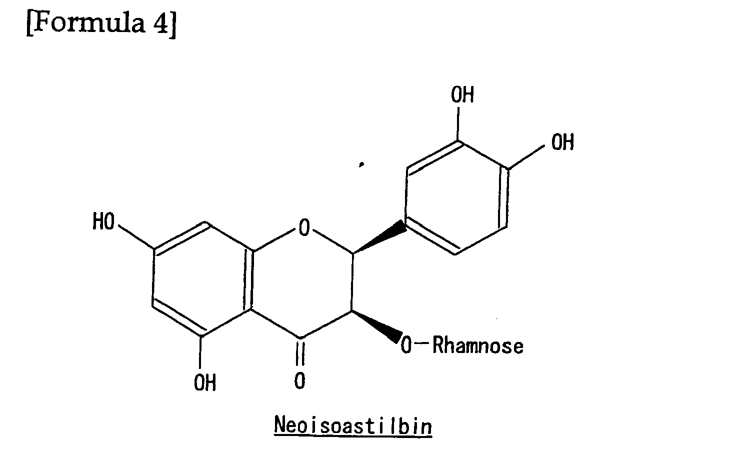

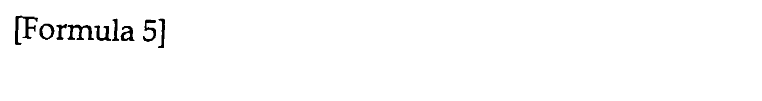

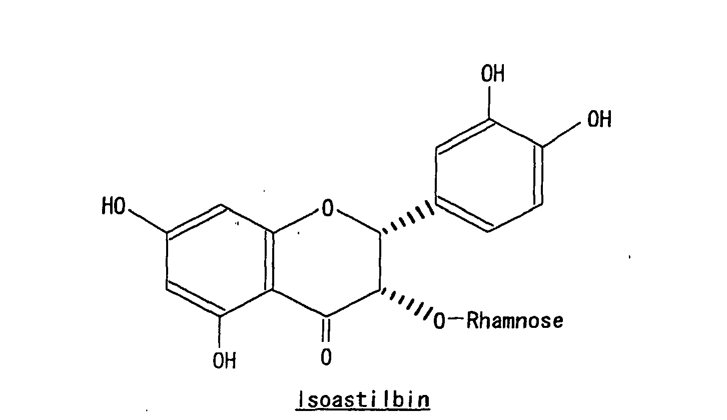

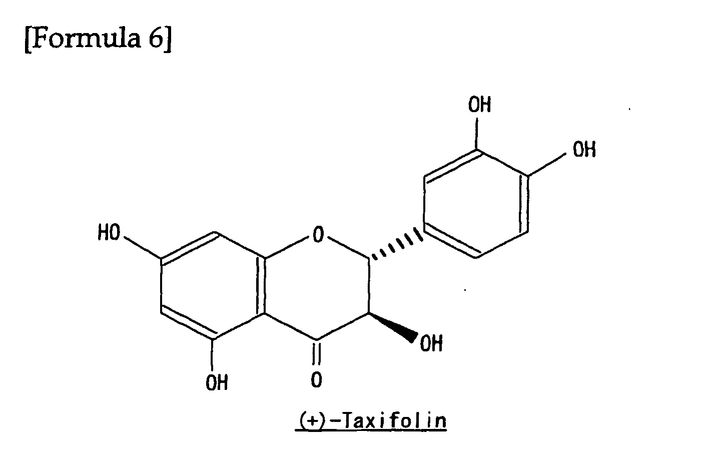

The dihydroflavonols of formula (1) above and glycosides thereof (particularly, astilbin, neoisoastilbin, isoastilbin, neoastilbin (hereinafter referred to as "astilbins"), taxifolin or any combination thereof) may be obtained by purification of extract of one or more plants selected from the group consisting of (but not limited to) *Engelhardtia chrysolepis, Lyonia ovalifolia, Chloranthus glaber, Smilax glabra, Astilbe microphylla* and *Astilbe odontophylla*. Among all, those prepared by purifying an extract of *Engelhardtia chrysolepis* leaves obtained by extraction with water, ethyl alcohol or mixture thereof may be preferable.

Particularly, solution of extracts prepared by extraction of the leaves of *Engelhardtia chrysolepis* with water-containing ethanol were then enriched, subjected to, for example, liquid-liquid countercurrent distribution method or column chromatography using ion-exchange resin or porous resin, and then purified to enrich for astilbins. Further, the enriched extract may be subjected to reversed-phase silica gel chromatography or normal phase silica gel chromatography, and optionally crystallized, to isolate astilbins.

Further, isolated astilbins may be treated with mineral acid (e.g., hydrochloric acid or sulfuric acid) or hydrolase (e.g., hesperidinase, naringinase, or anthocyanase) to hydrolyze astilbins to give taxifolin-enriched compounds.

In addition to such compounds isolated as described above, an extract of *Engelhardtia chrysolepis*, crude intermediate products produced at respective steps of the process, or any combination thereof may also be used in the therapeutic agents for improving bladder function or treating urinary disturbance which contain, as active ingredient, the dihydroflavonols of formula (1) above and/or glycosides thereof according the present invention.

For preparing formulation of an extract of *Engelhardtia chrysolepis*, carrier or any additive or additives (e.g., dextrin or cyclodextrin) which are commonly used for formulation may be added to facilitate preservation or handling thereof.

Therapeutic agents for improving bladder function or treating urinary disturbance according to the use of the present invention may be added directly to food and drink which may be then used to improve bladder function or treat urinary disturbance.

Thus obtained food and drink according to the use of the present invention has a great advantage that they can be taken easily as daily food and drink and thus may be expected to have great effects on bladder function and urinary disturbance. The term "food and drink" herein includes a variety of foods and drinks which can be orally administered, such as general foods, health foods, supplement foods, and quasi or pharmaceutical drugs.

The amount of therapeutic agent for improving bladder function or treating urinary disturbance which may be added to such food and drink may depend on the type of food or drink used. Such therapeutic agent may be added in any amount provided that the taste of the food or drink is not degraded. Therapeutic agent may typically be present at 0.01-50wt%, and preferably at 0.1-20wt% of the total food or drink. In the case of granule-, tablet- or capsule-type food, therapeutic agent may be present typically at 0.1-100wt%, and preferably 5-100wt% of the total food.

Suitable dose of the active ingredient contained in such therapeutic food or drink for improving bladder function or treating urinary disturbance according to the present invention may be about 1-3,000mg/adult/day though it can be modified depending on the age, condition or other factors of the patient.

Food and drink to which therapeutic agents for improving bladder function or treating urinary disturbance according to the use of the present invention are applicable include, but are not limited to: drinks such as soft drinks, carbonated beverages, nutritional drinks, fruit drinks or fermented lactic-drinks; frozen desserts such as ice cream, iced sorbet or crushed ice; noodles such as *soba, udon,* bean-starch vermicelli, shell of dumplings (such as *gyoza* or *shumai*), Chinese noodle or instant noodles; sweets such as barley sugar, candy, chewing gum, chocolate, tablet sweet, chips, biscuit, jelly, jam, cream, baked cake or bun; sea foods such as crab, salmon, *asari* clam, tuna, sardine, shrimp, bonito, mackerel, whale, oyster, saury, cuttlefish, ark shell, scallop, abalone, sea urchin, salmon roe or abalonel; processed marine or stock-farm products such as boiled fish paste, ham or sausage; dairy products such as processed or fermented milk; fats, fatty oils, and processed fats and fatty oils such as salad oil, oil for frying, margarine, mayonnaise, shortening, whipping cream or dressing; seasonings such as sauce or gravy; boil-in-the-bag type foods such as curry, stew, *oyako-don* (a bowl of rice with (soy-and-sugar-seasoned) chicken, egg and vegetables), rice gruel, a porridge of rice and vegetables, *Chuka-don*(a bowl of rice with a chop-suey-like mixture on it), *katsu-don* (a bowl of rice with a breaded and deep-fried pork cutlet on top), ten-don (a bowl of rice topped with deep-fried fish (and vegetables)), *una-don* (a bowl of eel and rice), *hayashi*-rice (rice with hashed meat), *oden* (vegetables, fish dumplings and various other articles of food stewed in a thin soy soup, and served hot), *mabo-dofu* (spicy tofu sauce covered rice bowl), *beef-don* (a bowl of rice with (soy-and-sugar-seasoned) beef and vegetables), meat sauce, egg soup, omelet with a filling of ketchup-seasoned fried rice, *gyoza* (a dumpling with minced pork and vegetable stuffing), *shumai* (steamed dumpling), hamburger steak or meat ball; various healthy or nutritional supplement foods; pharmaceutical or quasi drugs in a form of tablet, capsule, drink or lozenge. Optionally, any conventional subsidiary ingredient(s) and/or additive(s) may be added to such food and drink.

Such ingredients or additives include, but are not limited to, for example, glucose, fructose, sucrose, maltose, sorbitol, stevioside, rubusoside, corn syrup, lactose, citric acid, tartaric acid, malic acid, succinic acid, lactic acid, L-ascorbic acid, dl- a -tocopherol, sodium erythorbate, glycerine, propylene glycol, glycerine fatty acid ester, polyglycerine fatty acid ester, sucrose fatty acid ester, sorbitan fatty acid ester, gum arabic, carageenan, casein, gelatin, pectin, agar, vitamin B family, nicotinic acid amide, calcium pantothenate, amino acids, calcium salts, pigments, aromatics, and preservatives.

### Examples

Hereinafter, the present invention will be described in more detail in reference to the following Manufacture Examples and Examples. It should be noted, however, that the present invention will not be limited to these Examples.

### Manufacture Example 1: Preparation of an extract of Engelhardtia chrysolepis

The leaves of *Engelhardtia chrysolepis* were crushed. Next, 1000mL of extraction solvent, ethanol containing 30wt% water (ethanol: water=7:3 by weight) was added to 100g of the crushed leaves, and the mixture was gently stirred at 80°C for 2 hours and then filtered through a filter paper to obtain solution of an extract of *Engelhardtia chrysolepis*. Further, 1000mL of ethanol containing 30wt% water was added to the residue and the above process was repeated to obtain another solution of extract.

The two solutions were combined, concentrated under reduced pressure and then dried in a vacuum dryer to obtain dry product (yield = 26.2wt%).

Quantitative analysis of astilbins in the obtained extracts was performed by high-performance liquid chromatography. The results are shown in Table 1 below.

**[Table 1]**

| | Content in *Engelhardtia chrysolepis* (wt%) |
|---|---|
| astilbin | 11.60 |
| neoastilbin | 1.85 |
| isoastilbin | 0.83 |
| neoisoastilbin | 1.96 |

### Comparative Manufacture Example 2: Preparation of astilbin

Extract of *Engelhardtia chrysolepis* obtained in Manufacture Example 1 was suspended in 200mL of water and extracted with 200mL of ethyl acetate three times. Solution of extract obtained was concentrated to obtain ethyl acetate-extracted product (8.5g).

Next, 1g of the ethyl acetate-extracted product was separated on reversed-phase silica gel column by high-performance liquid chromatography to give purified astilbin (0.25g).

### Comparative Manufacture Example 3: Preparation of taxifolin

Extract of *Engelhardtia chrysolepis* obtained in Manufacture Example 1 was dissolved in 500mL of water, sterilized, and then incubated with 1g of crude hesperidinase (origin: *Aspergillus niger)* at 37°C for 24 hours to hydrolyze astilbins.

Solution of hydrolyzed astilbin was extracted with 200mL of ethyl ether three times, and ether phase was concentrated to give an extract (2.2g). The extract was then dissolved in 20mL of methanol, added with 5mL of water and left to stand at 5°C to obtain deposited taxifolin crystal.

Additional two rounds of filtration-recrystallization from methanol as described above gave purified taxifolin(0.9g).

### Example 1: Examination of bladder function in urination-obstructed rabbit model

Four groups of 2.5-3.0 kg New Zealand white rabbits (seven animals in each group) were prepared. Groups 1 and 2 daily received an extract of *Engelhardtia chrysolepis* obtained in Manufacture Example 1 via oral administration (100mg/kg/day) while groups 3 and 4 received distilled water in the same manner.

After 4 weeks, surgical operation was performed on the rabbits of groups 1 and 3 under pentobarbital anesthesia to cause urinary obstruction while sham-operation was performed on the rabbits of groups 2 and 4 without causing urinary obstruction. For 4 weeks after operation, administration was continued and bladder function was examined as described below. Further, bladders were removed and subjected to contraction test and biochemical assay as described below.

More particularly, surgical operation for inducing urinary obstruction was performed as follows: rabbits of groups 1 and 3 were anesthetized with pentobarbital (25mg/kg) and a catheter was introduced in their bladders. Abdominal cavity was opened such that bladder could be seen, and then fat around urinary tract was removed. Next, the urinary tract was tied up with 00 silk thread and then, after the catheter was removed, the wound was stitched up. After operation, the rabbits were contained in recovery rooms until they came out from the anesthetic while they were observed for 2- 3 hours. Animals were monitored daily to check for diet or water ingestion, urination behavior and the presence of pain. Nubain was administered (0.1mg/kg, im) to the animals in order to relieve pain (obstructive operation).

On the other hand, the same procedure was repeated for the rabbits of groups 2 and 4 except that urinary tract was not tied-up (sham-operation).

### <Examination of Bladder Function >

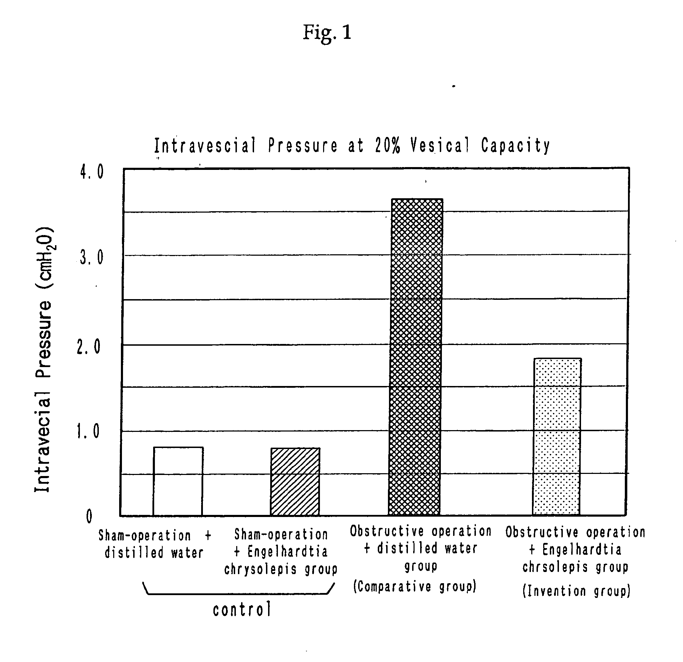

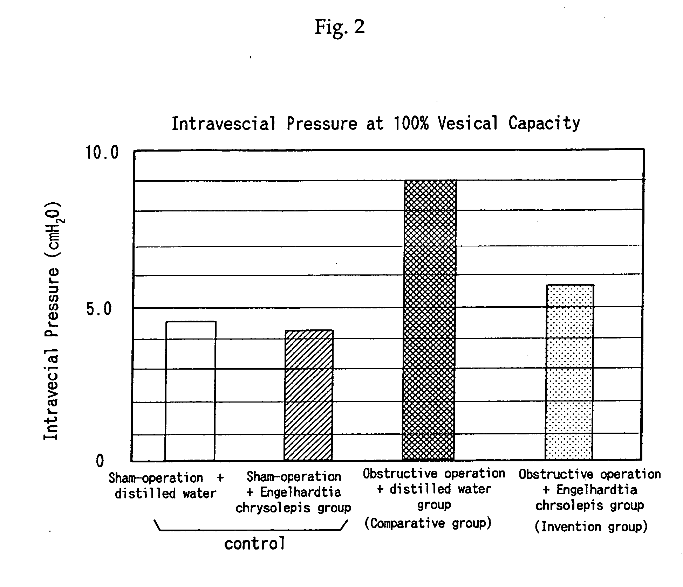

Before dissection, bladder function was examined in all of the 28 rabbits. Animals were anesthetized with ketamine-xylazine and a catheter was introduced into their bladders. A three-way cook was connected, at each end, to the catheter, an infusion pump, and a transducer for measuring intravesical pressure at excretion. After letting the animals to excrete urine through the catheter, physiological saline was injected to the animals at a rate of 1mL/min until micturition reaction occurred or urine was leaked (incontinence) from where the catheter was introduced, and intravesical pressure was recorded. Pressures at 20% and 100% vesical capacities are shown in Table 2 below and Figures 1 and 2.

**[Table 2]**

| | | Intravesical pressure at 20% vesical capacity (cmH₂O) | Intravesical pressure at 100% vesical capacity (cmH₂O) |
|---|---|---|---|
| Control | Sham-operation + distilled water | 0.81 | 4.49 |
| | Sham-operation + extract of *Engelhardtia chrysolepis* | 0.78 | 4.30 |
| Comparative group | Obstructive operation + distilled water | 3.64 | 8.89 |
| Invention group | Obstructive operation + extract of *Engelhardtia chrysolepis* | 1.81 | 5.80 |

It is apparent from the results shown in Table 2 and Figures 1 and 2 that the invention group exhibited lower intravesical pressures than those of the comparative group at both 20% and 100% vesical capacities, which shows that an extract of *Engelhardtia chrysolepis* may reduce intravesical pressure in urination-obstructed model. Therefore, such an extract can possibly keep elasticity of bladder detrusor (i.e., to prevent bladder hypofunction) and thus may be useful for improving bladder function.

### Example 2: Examination of Muscular Tissue Contraction

After intravesical pressure recorded, animals were anesthetized with pentobarbital and their bladders were then removed. After rabbits were euthanized, the bladders were opened to prepare bladder detrusor samples (15-20mm (length) × 5mm (thickness)).

The bladder detrusor samples were overhung in a Magnus device filled with 30mL of Tyrode's balanced salt solution (Composition: 124.9mM NaCl, 12.2mM KCl, 23.8mM NaHCO₃, 0.5mM MgCl₂, 0.4mM NaHPO₄, 1.8mM CaCl₂ and 5.5mM dextrose). The solution was aerated with 95% air, 5% carbon dioxide.

Each sample was connected to the transducer and contractive force was recorded on Grass Model 7D Polygraph as well as on an Pentium® supported Computer via A/D converter system. Each sample was trained with a tension of 2g for 30 minutes and electrically stimulated at 8Hz and 32Hz for 20 seconds to induce contraction. Then, carbachol (20 µM carbamylcholine chloride) was added to induce contraction again. Each sample was washed with fresh Tyrode's solution for 10 minutes three times during stimulation.

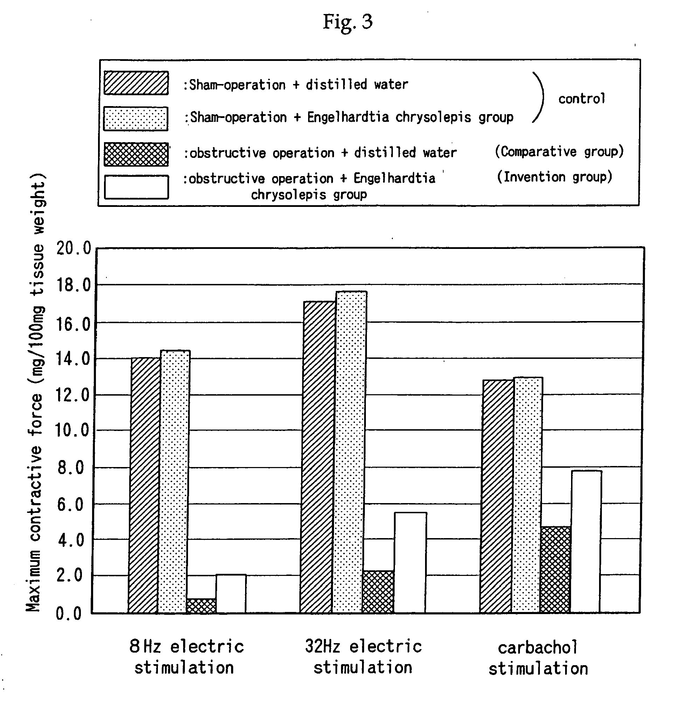

Maximum contractive force was calculated from the determined contraction for each sample using Polyview System. The results are shown in Table 3 and Figure 3.

**[Table 3]**

| | | Max Contractive force in response to electric or carbachol stimulation (mg/100mg tissue weight) | | |
|---|---|---|---|---|
| | | 8Hz | 32Hz | carbachol |
| Control | Sham-operation + distilled water | 14.1 | 17.1 | 12.8 |
| | Sham-operation + extract of *Engelhardtia chrysolepis* | 14.5 | 17.6 | 13.0 |
| Comparative group | Obstructive operation + distilled water | 0.8 | 2.3 | 4.7 |
| Invention group | Obstructive operation + extract of *Engelhardtia chrysolepis* | 2.1 | 5.5 | 7.8 |

It is apparent from the results shown in Table 3 and Figure 3 that the invention group exhibited higher maximum contractive forces than those of the comparative group in response to both electric or carbachol stimulation, which indicates that an extract of *Engelhardtia chrysolepis* has an ability to inhibit reduction in contractive force of ladder muscle force in response to electric or carbachol stimulation in urination-obstructed model. Accordingly, these results shows that such an extract may have an ability to inhibit hypofunction of neurotransmission to detrusor in urinary disturbance (i.e., to protect cholinergic nerve) and may be thus very effective in improving bladder function and treating urinary disturbance.

### Example 3: Biochemical Assay

### <Ca²⁺-dependent ATPase activity assay>

A slice of bladder (muscle and mucosa) was homogenized in 10mM Tris-malete, 10mM NaHCO₃(pH6.8). Homogenized solution was then filtered through a gauze, and centrifuged at 2000 X g for 12 minutes to obtain supernatant which was then centrifuged at 13300 × g for 30 minutes to remove mitochondria fraction. Centrifugation at 100000g for 60 minutes gave microsome fraction which was then dissolved in 10mM Tris-malete, 10 mM NaHCO₃ (pH6.8).

Obtained microsome solution was allowed to react in a tube containing Tris buffer in the co-presence of 1mM ATP and 3mM CaCl₂ at 37°C. Reaction was stopped by adding 1mL of 12.5% TCA to the solution.

The tube was centrifuged at 1000 X g for 20 minutes to precipitate protein. Inorganic phosphorus in supernatant was allowed to react with molybdic acid to produce molybdenum blue which was then assayed at 660nm. Inorganic phosphorus in ATP and tissue slice was calibrated and used for calculation. The results are shown in µmol Pi/mg protein/min.

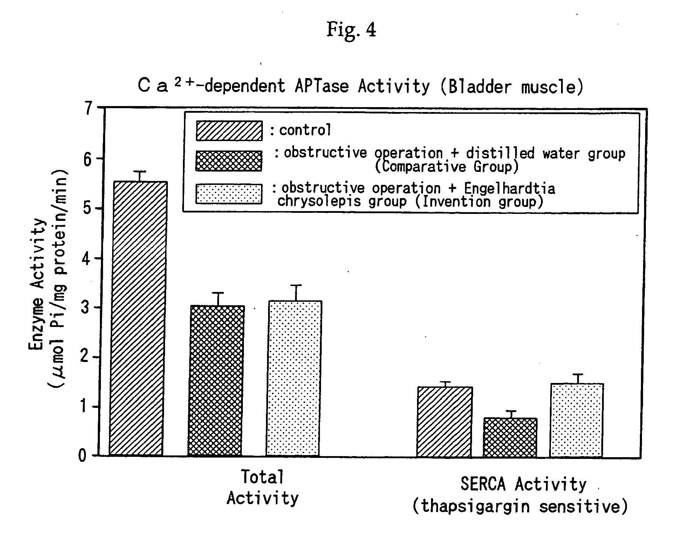

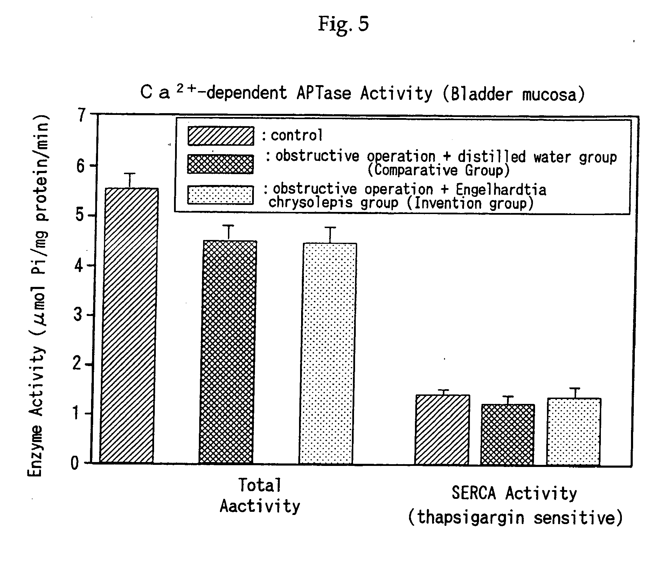

Next, Thapsigragin-inhibited SERCA activity was assayed by reaction in the presence or absence of 10 µM Thapsigragin. Total activity indicates the activity in the absence of Thapsigragin while SERCA activity indicates the difference between the activities in the presence and absence of Thapsigragin. The results obtained for bladder muscle and mucosa are shown in Figures 4 and 5, respectively.

It is apparent from Figure 4 that obstructed groups exhibited reduced total and SERCA activities of Ca²⁺-dependent ATPase when compared with those of the control group in bladder muscle. However, reduced SERCA activity induced by obstructive operation was improved by administration of extraction of *Engelhardtia chrysolepis*.

On the other hand, it is apparent from Figure 5 that obstructed groups showed slight reduction in the total enzyme activity in bladder mucosa but no reduction in the SERCA activity. These results were consistent with the results in the contractive reaction test in response to electric or carbachol stimulation

### <Citrate Synthase Activity Assay>

A slice of bladder (muscle and mucosa) was homogenized (20mg/mL) in cold physiological saline. Homogenized solution was then filtered through a gauze and 3 X or 6 X diluted for activity assay and protein quantification. Next, 100 µL of homogenate solution was loaded into a 0.5cm cuvette which contained 1mL of 0.05M Tris buffer (pH7.6), 50 µL of 10mM oxaloacetic acid, 30 µL of 12.3mM acetyl CoA, 100 µL of 1mM 5,5'-dithiobis-2-nitorobenzoic acid (DTNB) and 100 µL of 10% Triton X-100, and absorbance of reaction product at 415nm was determined. The determination was performed every 30 seconds for six minutes using an absorptiometer (HITACHI). Protein Quantification was performed according to Lowry process.

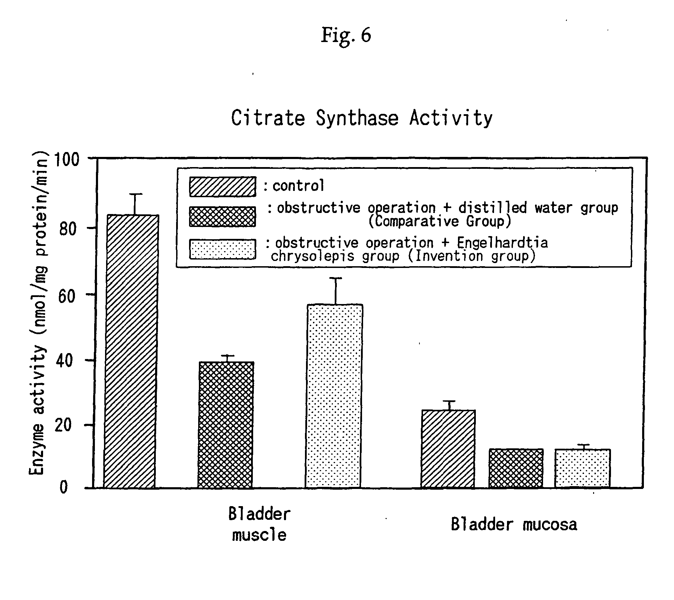

Results obtained for bladder muscle and mucosa are shown in Figure 6. Data are shown as mean ±standard error. Significant test was performed for each group based on Newman-Keul's test. The results were significant with p<0.01.

It is apparent from Figure 6 that both obstruction-distilled water and obstruction*-Engelhardtia chrysolepis* groups showed significant reduction in citrate synthase activity in bladder muscle and mucosa while obstruction*-Engelhardtia chrysolepis* group showed significant improvement of enzyme activity in bladder mucosa when compared with obstruction-distilled water group.

It is apparent from these results that an extract of *Engelhardtia chrysolepis* can be used to treat and prevent bladder dysfunction in rabbit by dramatically improving bladder function (intravesical pressure at miction), contractive reaction and biochemical function.

### Example 4: Tablet

Tablet was prepared in a conventional tablet-making machine using the following ingredients:

| | |
|---|---|
| powdery extract of *Engelhardtia chrysolepis* | |
| obtained in Manufacture Example 1 | 20 parts |
| dextrin | 72 parts |
| powdered sugar | 80 parts |
| glycerin fatty ester | 8 parts. |

Simple process of mixing ingredients and tablet-making provided a tasty tablet which was effective in improving bladder function and treating urinary disturbance.

### Comparative Example 5: Tablet

Tablet was prepared in a conventional tablet-making machine using the following ingredients:

| | |
|---|---|
| astilbin powder obtained in Manufacture Example 2 | 10 parts |
| taxifolin powder obtained in Manufacture Example 3 | 10 parts |
| dextrin | 72 parts |
| powdered sugar | 80 parts |
| glycerin fatty ester | 8 parts. |

Simple process of mixing ingredients and tablet-making provided a tasty tablet which was effective in improving bladder function and treating urinary disturbance.

### Comparative Example 6: Tablet

Tablet was prepared in a conventional tablet-making machine using the following ingredients:

| | |
|---|---|
| powdery extract of *Engelhardtia chrysolepis* | |
| obtained in Manufacture Example 1 | 15 parts |
| taxifolin powder obtained in Manufacture Example 3 | 5 parts |
| dextrin | 72 parts |
| powdered sugar | 80 parts |
| glycerin fatty ester | 8 parts. |

Simple process of mixing ingredients and tablet-making provided a tasty tablet which was effective in improving bladder function and treating urinary disturbance.

### Example 7: Capsule

Capsule was prepared by encapsulating the following ingredients in hard gelatin capsule #1 according to a conventional method:

### <Composition of capsule per unit dose (200mg)>

| | |
|---|---|
| powdery extract of *Engelhardtia chrysolepis* | |
| obtained in Manufacture Example 1 | 5 mg |
| corn starch | 60.0mg |
| lactose | 100.0mg |
| calcium lactate | 10.0mg |
| hydroxypropyl cellulose (HPC-L) | 10.0mg |

### Comparative Example 8: Capsule

Capsule was prepared by encapsulating the following ingredients in hard gelatin capsule #1 according to a conventional method:

| | |
|---|---|
| <Composition of capsule per unit dose (200mg)> | |
| astilbin powder obtained in Manufacture Example 2 | 3mg |
| taxifolin powder obtained in Manufacture Example 3 | 3mg |
| corn starch | 60.0mg |
| lactose | 100.0mg |
| calcium lactate | 10.0mg |
| hydroxypropyl cellulose (HPC-L) | 10.0mg |

### Example 9: Powdery instant tea

Powdery instant teas were prepared in a fluid-bed granulating machine using the following ingredients:

| | |
|---|---|
| powdery extract of *Engelhardtia chrysolepis* | |
| obtained in Manufacture Example 1 | 20 parts |
| oligosaccharide | 40 parts |
| citric acid | 50 parts |
| sugar | 50 parts |
| dextrin | 810parts |

Simple process of mixing and granulating ingredients provided tasty powdery instant teas which was effective in improving bladder function and treating urinary disturbance.

### Comparative Example 10: Powdery instant tea

Powdery instant teas were prepared in a fluid-bed granulating machine using the following ingredients:

| | |
|---|---|
| astilbin powder obtained in Manufacture Example 2 | 10 parts |
| taxifolin powder obtained in Manufacture Example 3 | 10 parts |
| oligosaccharide | 40 parts |
| citric acid | 50 parts |
| sugar | 50 parts |
| dextrin | 810 parts |

Simple process of mixing and granulating ingredients provided tasty powdery instant teas which was effective in improving bladder function and treating urinary disturbance.

### Example 11: Biscuit

A dough was prepared by mixing 1 kg of flour, 100g of corn starch, 250g of sugar, 125g of margarine, 5g of salt, 25g of sodium carbomate, 9g of ammonium carbonate, 6g of lecithin, 75g of whole egg, 50g of calcium lactate, 2g of powdery extract of *Engelhardtia chrysolepis* obtained in Manufacture Example 1 and 350g of water. The dough was then rolled out, molded into shapes and baked to obtain biscuits which had good taste and an efficient ability to improve bladder function and treat urinary disturbance.

### Comparative Example 12: Biscuit

A dough was prepared by mixing 1 kg of flour, 100g of corn starch, 250g of sugar, 125g of margarine, 5g of salt, 25g of sal soda, 9g of ammonium carbonate, 6g of lecithin, 75g of egg, 50g of calcium lactate, 1g of astilbin powder obtained in Manufacture Example 2, 1g of taxifolin powder obtained in Manufacture Example 3 and 350g of water, spread, formed and baked to obtain biscuits which had good taste and an efficient ability to improve bladder function and treat urinary disturbance.

### Example 13: Jelly candy (gumi candy)

Mixture of 330 g of palatinose, 140g of sorbitol and 270g of water was heated and added with solution of 80g of gelatin and 10g of powdery extract of *Engelhardtia chrysolepis* obtained in Manufacture Example 1 in 150g of water, and with 8g of 50% aqueous phosphate solution. The mixture was then poured into a mould and cooled to provide tasty jelly candy which is effective in improving bladder function and treating urinary disturbance.

### Comparative Example 14: Jelly candy (gumi candy)

Mixture of 330 g of palatinose, 140g of sorbitol and 270g of water was heated and added with solution of 80g of gelatin, 5g of astilbin powder obtained in Manufacture Example 2 and 5g of taxifolin powder obtained in Manufacture Example 3 in 150g of water and with 8g of 50% aqueous phosphate solution. The mixture was then poured into a mould and cooled to obtain tasty jelly candy which was effective in improving bladder function and treating urinary disturbance.

### Example 15: Chewing gum

Gum base (25 parts) and palatinit syrup (14 parts) were mixed in a chewing gum mixer arranged for trial. Additionally, premix of oligosaccharide (35 parts), sugar (25 parts), powdery extract of *Engelhardtia chrysolepis* obtained in Manufacture Example 1 (14 parts) and stevia (Maruzen Pharmaceuticals Co., Ltd. Trade Mark "Marumiron 50" registered in Japan) (0.4 parts) was added in portions to the mixer while kneading.

Then, glycerin (1 part) was added to the mixer and, after sufficient mixing, the mixture was removed from the mixer and rolled out to give a tasty chewing gum which was effective in improving bladder function and treating urinary disturbance.

### Comparative Example 16: Chewing gum

Gum base (25 parts) and palatinit syrup (14 parts) were mixed in a chewing gum mixer arranged for trial. Additionally, premix of oligosaccharide (35 parts), sugar (25 parts), astilbin powder (8 parts) obtained in Manufacture Example 2, taxifolin powder (6 parts) obtained in Manufacture Example 3 and stevia (Maruzen Pharmaceuticals Co., Ltd. Trade Mark "Marumiron 50" registered in Japan) (0.4 parts) was added in portions to the mixer while kneading.

Then, glycerin (1 part) was added to the mixer and, after sufficient mixing, the mixture was removed from the mixer and rolled out to give a tasty chewing gum which was effective in improving bladder function and treating urinary disturbance.

### Example 17: Mouth wash

Mouth wash was prepared according to a conventional method using the following ingredients:

| | |
|---|---|
| ethanol | 15 parts |
| oligosaccharide | 10 parts |
| citric acid | 0.05 parts |
| sodium citrate | 0.2 parts |
| sodium benzoate | 0.2 parts |
| sodium lauryl sulfate | 0.2 parts |
| sodium saccharin | 0.05 parts |
| extract of *Engelhardtia chrysolepis* obtained in Manufacture Example 1 | 0.4 parts |
| 1-menthol | 0.05 parts |
| purified water Total | the reminder 100 parts |

Mouth wash prepared had good taste and an efficient ability to improve bladder function and treat urinary disturbance.

## Claims

1. The use of an extract of *Engelhardtia chrysolepis* as active ingredient for the production of a therapeutic agent for improving bladder function or treating urinary disturbance.

2. The use according to claim 1 **characterized in that** the extract of *Engelhardtia chrysolepis* is obtained by extracting the leaves and/or stalk of *Engelhardtia chrysolepis* or the whole *Engelhardtia chrysolepis* with water, hydrophilic organic solvent or mixture thereof.

3. The use according to claim 1 or 2, **characterized in that** the extract of *Engelhardtia chrysolepis* is obtained by extracting leaves of *Engelhardtia chrysolepis* with water, ethyl alcohol or mixture thereof.

4. The use of the active ingredient recited in any of claims 1 to 3 for the preparation of a food and/or drink effective in improving bladder function or treating urinary disturbance.

5. The use according to claim 4 **characterized in that** the active ingredient recited in any of claims 1 to 3 is used in an amount of 0.01-50 % by weight of the total food or drink.

6. The use according to claim 4 **characterized in that** the active ingredient recited in any of claims 1 to 3 is used in an amount of 0.1-100 % by weight of the total food or drink and that the food or drink is provided in a form of granules, tablets or capsules.

## Patentansprüche

1. Verwendung eines Extrakts aus *Engelhardtia chrysolepis* als aktiver Inhaltsstoff für die Herstellung eines therapeutischen Mittels zum Verbessern der Blasenfunktion oder zur Behandlung von Störungen des Urinaltrakts.

2. Verwendung gemäß Anspruch 1, **dadurch gekennzeichnet, dass** der Extrakt aus *Engelhardtia chrysolepis* durch Extrahieren der Blätter und/oder des Stengels von *Engelhardtia chrysolepis* oder der gesamten Pflanze *Engelhardtia chrysolepis* mit Wasser, hydrophilem organischem Lösungsmittel oder einer Mischung daraus erhalten wird.

3. Verwendung gemäß Anspruch 1 oder 2, **dadurch gekennzeichnet, dass** der Extrakt aus *Engelhardtia chrysolepis* durch Extrahieren der Blätter von *Engelhardtia chrysolepis* mit Wasser, Ethylalkohol oder einer Mischung daraus erhalten wird.

4. Verwendung des aktiven Inhaltsstoffs gemäß einem der Ansprüche 1 bis 3 zur Herstellung eines Nahrungsmittels und/oder eines Getränks, das wirksam darin ist, die Blasenfunktion zu verbessern oder Störungen des Urinaltrakts zu behandeln.

5. Verwendung gemäß Anspruch 4, **dadurch gekennzeichnet, dass** der aktive Inhaltsstoff gemäß einem der Ansprüche 1 bis 3 in einer Menge von 0,01 bis 50 Gew.-% des gesamten Nahrungsmittels oder Getränks verwendet wird.

6. Verwendung gemäß Anspruch 4, **dadurch gekennzeichnet, dass** der aktive Inhaltsstoff gemäß einem der Ansprüche 1 bis 3 in einer Menge von 0,1 bis 100 Gew.-% des gesamten Nahrungsmittels oder Getränks verwendet wird und dass das Nahrungsmittel oder das Getränk in der Form von Granulat, Tabletten oder Kapseln zur Verfügung gestellt wird.

## Revendications

1. Utilisation d'un extrait de *Engelhardtia chrysolepis* en tant qu'ingrédient actif pour la production d'un agent thérapeutique destiné à améliorer la fonction de la vessie ou pour traiter une perturbation urinaire.

2. Utilisation selon la revendication 1, **caractérisée en ce que** l'extrait de *Engelhardtia chrysolepis* est obtenu par extraction des feuilles et/ou de l'écorce de *Engelhardtia chrysolepis* ou de *Engelhardtia chrysolepis* entière avec de l'eau, un solvant organique hydrophile ou un de leurs mélanges.

3. Utilisation selon la revendication 1 ou 2, **caractérisée en ce que** l'extrait de *Engelhardtia chrysolepis* est obtenu par extraction de feuilles de *Engelhardtia chrysolepis* avec de l'eau, de l'alcool éthylique ou un de leurs mélanges.

4. Utilisation de l'ingrédient actif indiqué dans l'une quelconque des revendications 1 à 3, pour la préparation d'un aliment et/ou d'une boisson efficace pour améliorer la fonction de la vessie ou traiter une perturbation urinaire.

5. Utilisation selon la revendication 4, **caractérisée en ce que** l'ingrédient actif indiqué dans l'une quelconque des revendications 1 à 3, en une quantité de 0,01 à 50 % en poids de l'aliment ou boisson total.

6. Utilisation selon la revendication 4, **caractérisée en ce que** l'ingrédient actif indiqué dans l'une quelconque des revendications 1 à 3 est utilisé en une quantité de 0,1 à 100 % en poids de l'aliment ou boisson total, et **en ce que** l'aliment ou boisson se présente sous la forme de granules, comprimés ou capsules.
